# EUROPEAN PATENT APPLICATION

(11) **EP 4 830 646 A2**
(43) Date of publication of application: **28.10.2026**
(21) Application number: 26199532.8
(22) Date of filing: 15.12.2021
(51) Int. Cl.: A61B 17/12, A61M 37/00, A61B 18/14, A61B 5/00

(54) **A DEVICE TO OCCLUDE A BODY LUMEN**

(30) Priority: 15.12.2020 EP 20214367
(62) Document number(s) of the earlier application(s) in accordance with Art. 76 EPC: 26178825.1; 21836542.7 / 4 262 582
(71) Applicant: Aurigen Medical Limited, H91 DCH9 Galway (IE)
(72) Inventor: O'HALLORAN, Tony, Galway, H65 YK26 (IE); THOMSON, John, Dublin, D13 K039 (IE); KELLY, John, Galway, H91 NP2W (IE); MORAN, Matt, Co. Galway, H91 (IE)
(74) Representative: Purdylucey Intellectual Property

(57) **Abstract**

A device to occlude a body lumen such as the left atrial appendage of a heart of a subject is described. The device comprises an implantable occlusion apparatus comprising a proximal connecting hub and a radially expansible body configured for radial expansion upon deployment from a contracted configuration to a radially expanded configuration to fluidically occlude the left atrial appendage, an elongated deployment catheter having a central lumen, an elongated delivery catheter disposed in the central lumen of the elongated deployment catheter and having a distal connecting hub detachably attachable to the proximal connecting hub of the implantable occlusion apparatus for transluminal delivery of the implantable occlusion apparatus to the left atrial appendage, wherein the elongated deployment catheter is axially movable proximally relative to the elongated delivery catheter to deploy the implantable occlusion apparatus. The device comprises an anchoring module comprising a circumferential array of anchoring arms configured for adjustment from (a) a delivery configuration in which the array of anchoring arms are arranged axially and (b) a deployed configuration in which the anchoring arms are splayed radially outwardly to engage a wall of the left atrial appendage through an open section of a sidewall of the occlusion apparatus.

## Description

### Field of the Invention

The present invention relates to a device to occlude a body lumen, in particular a left atrial appendage (LAA) of the heart. The invention also relates to a method of occluding a body lumen such as the left atrial appendage of the heart.

### Background to the Invention

Atrial fibrillation (AF) is a common cardiac rhythm disorder affecting an estimated 6 million patients in the United States alone. AF is the second leading cause of stroke in the United States and may account for nearly one-third of strokes in the elderly. As our population continues to age, this problem may become even more prevalent. In greater than 90% of cases where a blood clot (thrombus) is found in the AF patient, the clot develops in the left atrial appendage (LAA) of the heart. The irregular heart beat in AF causes blood to pool in the left atrial appendage, because clotting occurs when blood is stagnant, clots or thrombi may form in the LAA. These blood clots may dislodge from the left atrial appendage and may enter the cranial circulation causing a stroke, the coronary circulation causing a myocardial infarction, the peripheral circulation causing limb ischemia, as well as other vascular beds. The LAA is a muscular pouch of heart attached to the left atrium. Mechanical occlusion of the LAA may result in a reduction of the incidence of stroke in AF patients, and there is growing interest in both surgical and endovascular methods to remove isolate the LAA.

Anti-clotting drugs may be used to prevent strokes in patients diagnosed with AF. However, many people cannot take such drugs because of potential side effects. Drug therapy may also cause bleeding and may be difficult to control because determining dosage is challenging. Recent studies indicate that elimination of the LAA, through occlusion or closure, may prevent thrombi from forming in the LAA and thus may reduce the incidence of stroke in patients diagnosed with AF. As such, occlusion or closure of the LAA may significantly reduce the incidence of stroke in patients with atrial fibrillation and without the complications of drug therapy.

Device for occlusion of the left atrial appendage (LAA) of the heart are described in for example, EP3606448, EP 3606447, US2015/0196300 and WO2013/067118. The devices include a delivery catheter and a deployable radially expandable occlusion apparatus detachably attached to the occlusion apparatus. The devices are advanced transluminally through the vasculature to position the occlusion apparatus in the LAA, whereupon the occlusion apparatus is deployed to circumferentially engage the wall of the LAA and fluidically occlude the LAA. The wall of the LAA may then be treated using tissue mapping and ablation electrodes attached to the deployed occlusion apparatus to electrically isolate the LAA and thereby treat atrial fibrillation.

The LAA occlusion device generally include anchoring elements as part of the radially expansible occlusion apparatus that deploy with the occlusion apparatus. In the devices of US2015/0196300 and WO2013/067118, the anchoring elements comprise barbs attached to the expansible cage at the distal end of the cage. When the cage is deployed into engagement with the wall of the LAA, the barbs engage the wall of the LAA at the same time as the cage engages the wall, fixing the cage to the wall. In addition, the barbs engage the wall of the LAA distal to the cage and therefore distal to part of the wall LAA that is ablated during treatment.

US2018/0250014 describes a device for occlusion of a body lumen comprising a tubular foam body and a compliant cage disposed within the tubular foam body. In one embodiment, the compliant cage comprises anchoring barbs that project through the tubular foam body upon deployment. As the barbs form part of the cage frame, the barbs are connected together and deploy together as the cage frame deploys.

It is an object of the invention to overcome at least of the above-referenced problems.

### Summary of the Invention

The Applicant has realised that the devices of the prior art, in which the anchoring elements are connected together as part of a radially deployable cage structure, is not ideal for anchoring in non-uniform structures like the Left Atrial Appendage (LAA). This is because the anchor elements deploy together with the cage and upon deployment are arranged along a circumference of the cage in a predetermined (generally uniform) shape. The Applicant has addressed this problem by providing a circumferential array of anchoring arms that are connected to and extend distally from a proximal hub of the occlusion apparatus, where the arms are configured for pivotable self-adjustment from a delivery configuration in which the array of anchoring arms are arranged generally axially and a deployed configuration in which the anchoring arms are splayed radially outwardly to engage a wall of the left atrial appendage through an open section of a sidewall of the occlusion apparatus. As the arms can deploy independently of each other (and are generally not connected to the radially expansible body), the design allows the arms engage LAA's that have a non-uniform shape where the arms splay radially outwardly independently of each other to adapt to non-uniform LAA anatomies (e.g. self-adjusts). This is suitable for LAA anatomies that include wall invaginations where the radially expansible body does not engage the invaginated section of the wall but the array of arms can self-adjust to circumferentially engage the wall of the LAA including invaginated sections.

In addition, the Applicant has realised that there is a benefit to being able to deploy the occlusion apparatus partially into engagement with the wall of the LAA, while the anchoring barbs are not yet fully deployed and not in engagement with the tissue. This allows a user to partially deploy the occlusion apparatus into engagement with the wall of the LAA, check the positioning and re-position the device if required (or perform an initial treatment step), before fully deploying the device so that the anchoring barbs engage the tissue. This is achieved by providing a device in which the anchoring module is separate from the radially expandable occlusion body, allowing the deployment of each to be separately controlled. In one embodiment, the device is configured for deployment in at least two steps, a first partial deployment step in which the occlusion apparatus is radially expanded to engage tissue and the anchoring arms are not fully deployed, and a second deployment step in which the anchoring arms are fully deployed to engage tissue and anchoring the occlusion apparatus. In one embodiment of the device described herein, the occlusion apparatus has a proximal hub part and a radially expansible part, and the anchoring arms are attached to and extend distally from the proximal hub and are movable independently of the radially expansible part, providing flexibility to allow the radially expansible part (e.g. mesh cage) to be deployed into engagement with tissue before the anchoring barbs are fully deployed. Delaying full deployment of the anchoring arms may be achieved by shaping the arms, especially a proximal end of the arms, to cooperate with the occlusion apparatus during deployment so that they only fully deploy when the occlusion apparatus is nearly fully, or fully, deployed. Other methods of delaying deployment of the anchoring arms relative to deployment of the radially expansible part are described herein.

In a first aspect, the invention relates to a device to occlude a body lumen such as a left atrial appendage of a heart of a subject, comprising:
an implantable occlusion apparatus configured for radial expansion upon deployment from a contracted configuration to a radially expanded configuration to fluidically occlude the left atrial appendage;
an elongated delivery catheter having a distal connecting hub attachable to the implantable occlusion apparatus for transluminal delivery of the implantable occlusion apparatus to the left atrial appendage;
characterised in that the device comprises an anchoring module comprising a circumferential array of anchoring arms configured for adjustment from
(a) a delivery configuration in which the array of anchoring arms are arranged generally axially and
(b) a deployed configuration in which the anchoring arms are splayed radially outwardly to engage a wall of the left atrial appendage through an open section of a sidewall of the occlusion apparatus.

This device of the invention may also be employed to capture embolus in the blood stream. In such embodiments, the occlusion apparatus may be replaced with an embolus capture apparatus designed to filter blood and capture and retain embolus in the blood that passes through the embolus capture apparatus. The embolus capture apparatus may be a cage with a mesh size configured to allow passage of blood but to retain embolus of a defined minimum size, The embolus capture apparatus may also be configured to fluidically occlude a blood vessel.

In another aspect, the invention relates to a device for capturing embolus in a blood vessel, comprising:
an implantable embolus capture apparatus configured for radial expansion upon deployment from a contracted configuration to a radially expanded configuration;
an elongated delivery catheter having a distal connecting hub attachable to the implantable embolus capture apparatus for transluminal delivery of the implantable embolus capture apparatus to a target blood vessel.
characterised in that the device comprises an anchoring module comprising a circumferential array of anchoring arms configured for adjustment from
   (a) a delivery configuration in which the array of anchoring arms are arranged generally axially and
   (b) a deployed configuration in which the anchoring arms are splayed radially outwardly to engage a wall of the blood vessel through a sidewall of the implantable embolus capture apparatus.

In any embodiment, the device comprises an elongated deployment catheter having a central lumen in which the elongated delivery catheter is disposed in the central lumen of the elongated deployment catheter, wherein the elongated deployment catheter is axially movable proximally relative to the elongated delivery catheter to deploy the implantable occlusion apparatus or the implantable embolus capture apparatus.

In any embodiment, the implantable occlusion apparatus or the implantable embolus capture apparatus comprises a proximal connecting hub and a radially expansible body configured for radial expansion upon deployment from a contracted configuration to a radially expanded configuration.

In any embodiment, the anchoring arms are movable independently of (e.g. not attached to) the radially expansible body.

In any embodiment, the anchoring arms are attached to and extend distally from the proximal connecting hub.

In any embodiment, the anchoring arms are adjustable from the delivery configuration to the deployed configuration independently of each other.

In any embodiment, the anchoring arms are configured to pivot radially outwardly about their proximal end upon deployment.

In any embodiment, the anchoring arms are self-adjustable from the delivery configuration to the deployed configuration.

Each anchoring arm generally has a proximal end connected to the connecting hub. The connection generally allows articulation of the arm from a generally axial configuration to a radially outwardly angled configuration. The anchoring arms are generally movable independently of each other, allowing some arms to be angled outwardly more than others. This enables the arms of the anchoring module to self-adjust to the anatomy of the body lumen in which the device is located.

In any embodiment, the anchoring arms are directly connected to the proximal hub of the occlusion apparatus or embolus capture apparatus.

In another embodiment, the anchoring arms are indirectly connected to the proximal hub. The anchoring module may comprise an anchoring module hub configured for detachable attachment to the proximal hub of the occlusion body. The anchoring module may be movable axially relative to the occlusion apparatus or capture apparatus. This embodiment allows an occlusion apparatus/capture apparatus to be delivered to a target location separately from the anchoring module, and also allows an anchoring module to be recaptured and withdrawn prior to withdrawal of the occlusion apparatus/capture apparatus.

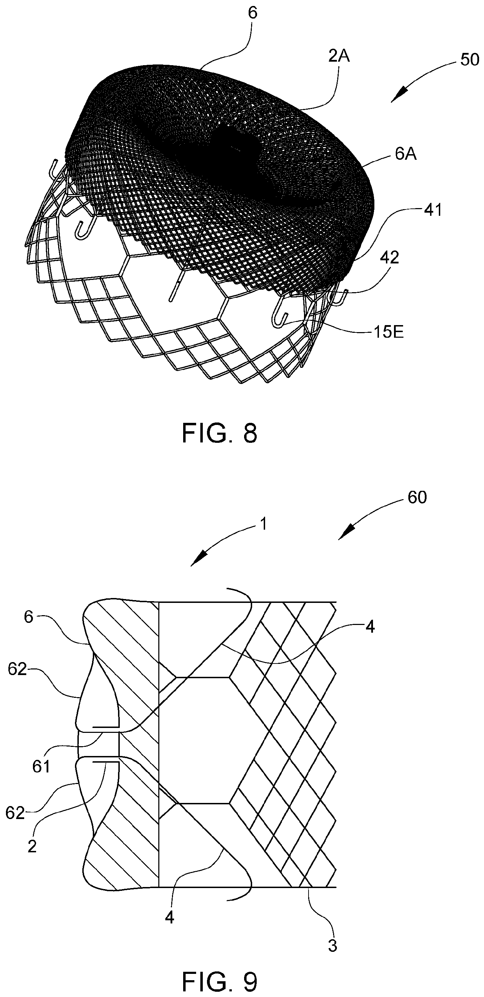

In any embodiment, the anchoring module hub comprises a proximal cover element configured to abut a proximal face of the radially expansible body when the anchoring module hub is attached to the proximal hub of the occlusion apparatus or capture apparatus. In any embodiment, the proximal face of the radially expansible body is concave and the proximal hub is disposed in a distally recessed part of the proximal face. In any embodiment, the proximal cover element may be configured to fluidically seal against the proximal face of the radially expansible body. In any embodiment, the proximal cover element may be configured to fluidically occlude the proximal hub when the anchoring module hub and proximal hub are attached together. The proximal cover is generally a planar element. The proximal cover may be formed of a liquid impermeable material. The proximal cover may be attached to a distal periphery of the anchoring module hub and extend radially outwardly of the anchoring module hub. An anchoring module comprising an anchoring module hub and proximal cover element is illustrated in Figure 9.

In any embodiment, the device is configured for adjustment from a partially deployed configuration in which the sidewall of the occlusion apparatus or capture apparatus engages a wall of the body lumen and the anchoring arms are not engaged with the wall of the body lumen and a fully deployed configuration in which the anchoring arms are engaged with the wall of the body lumen to anchor the occlusion apparatus or capture apparatus in the body lumen.

In any embodiment, one or more and generally all of the anchoring arms has a proximal section and a distal section, in which the proximal section has an inflection zone (e.g. a shoulder) configured to cooperate with the proximal end of the radially expansible body during deployment to deploy the device into the partially deployed configuration and upon further deployment into the fully deployed configuration. This allows the radially expansible element be deployed into engagement with the wall of the body lumen before the anchoring arms engage the wall.

In any embodiment, the inflection zone of the arm comprises an s-shaped section having a proximal inwardly curved part and a distal outwardly curved part.

In any embodiment, the anchoring module is configured for self-deployment upon deployment of the implantable occlusion apparatus or capture apparatus.

In any embodiment, each anchoring arm in a deployed configuration is splayed radially outwardly at an angle of 30-80° to a central axis of the implantable occlusion apparatus or capture apparatus.

In any embodiment, the anchoring arms have an axial length that is less than 70% of an axial length of the cage.

In any embodiment, the anchoring arms have an axial length that is 70% to 130% of an axial length of the cage.

In any embodiment, the radially expansible body comprises a mesh cage.

In any embodiment, the sidewall of the mesh cage has a proximal part, a distal part, and an intermediate part having a mesh size greater than a mesh size of the distal or proximal parts.

In any embodiment, the intermediate part of the mesh cage comprises one or more struts that project radially outwardly of the sidewall of the mesh cage.

In any embodiment, the one or more struts that project radially outwardly of the sidewall of the mesh cage comprise a tissue treatment electrode.

In any embodiment, the proximal end of the occlusion apparatus or capture apparatus has a recessed base and the proximal connecting hub is disposed in the recessed base.

In any embodiment, a distal end of one or more of the anchoring arms comprises an anchoring barb configured to engage tissue.

In any embodiment, the anchoring barb is curved radially outwardly In any embodiment, the anchoring barb is curved radially outwardly and proximally.

In any embodiment, the anchoring barb has a distal section that extends proximally parallel to a longitudinal axis of the occlusion device.

In any embodiment, the anchoring barb comprises two or more forks and in one embodiment is bifurcated.

In any embodiment, the anchoring barb bifurcates to provide a distal barb part and a proximal barb part.

In any embodiment, the anchoring barb bifurcates laterally.

In any embodiment, the bifurcation of the anchoring barb is axially angled to enhance the ability of the barbs to engage with invaginations of the wall of the body lumen (e.g. LAA ostium wall).

In any embodiment, the anchoring barb comprises a non-slip material or coating. This may be an anti-slip micro or nano structure material, to enhance anti-migration while minimising tissue injury.

In any embodiment, the anchoring barb comprises a contrast agent to enhance visualisation during imaging, for example fluoroscopic imaging.

In any embodiment, one or more of the anchoring barbs are coated with a pharmaceutically active agent.

In any embodiment, one or more of the anchoring barbs comprises a tissue parameter sensor. The sensor may detect any tissue parameter, for example temperature, blood flow, pH, electrical activity.

In any embodiment, one or more of the anchoring arms may comprise a lumen for delivery of a fluid to the wall of the LAA. The delivery catheter may include a lumen for delivery of a fluid to the anchoring arms, and the lumen may be configured to fluidically connected to the one or more anchoring arms with a lumen. The fluid may be a therapeutically active agent, or a diagnostic reagent; examples include a drug, contrast agent, or alcohol for tissue ablation.

In any embodiment, the distal section of at least one of the anchoring arms is cranked intermediate its ends such that when the anchoring arm is deployed an inner section is angled radially outwardly and an outer section extends parallel to a longitudinal axis of the occlusion apparatus.

In any embodiment, the anchoring module is configured for axial movement relative to the occlusion apparatus from a position distal of the occlusion apparatus to a position within the occlusion apparatus.

In any embodiment, the occlusion apparatus comprises a central proximal hub with a hollow lumen and a radially expansible body connected to the central proximal hub, wherein the axially movable anchoring module is configured for axial movement through the central lumen of the central proximal hub.

In any embodiment, the device comprises a tissue treatment or diagnosis module. The tissue treatment module may be configured to treat the tissue electrically, by cryogenic treatment, by microwave treatment, or RF energy treatment. The tissue diagnosis module may be configured to map the electrical activity of the body lumen or adjacent structures.

In any embodiment, the treatment module comprises an electrode. This electrode may comprise of a carbon-based material, graphite, graphene, or a carbon nano structures to enhance structural and operational functionality.

In any embodiment, the treatment module comprises an array of electrodes.

In any embodiment, the treatment module comprises a circumferential array of electrodes.

In any embodiment, the treatment module comprises a circumferential array of electrodes attached to and deployable with the occlusion apparatus.

In any embodiment, the device comprises a handle including actuating means to deploy occlusion apparatus and anchoring module.

In any embodiment, the actuation means of the handle is configured control deployment of the anchoring module independently of the deployment of the occlusion apparatus.

In any embodiment, the actuation means of the handle is configured to pause deployment of the anchoring module in a partially deployed configuration while the occlusion apparatus (e.g. radially expansible body) is fully or nearly fully deployed.

In another aspect, the invention provides a method of fluidically occluding a body lumen comprising the steps of:
providing a device according to the invention with the occlusion apparatus and delivery catheter disposed within the deployment catheter;
advancing the device of the invention transluminally until a distal end of the device is disposed in the body lumen;
deploying the device by retracting the deployment catheter relative to the delivery catheter to deploy the occlusion apparatus and anchoring arms;
detaching the delivery catheter from the occlusion apparatus; and
retracting the delivery catheter and deployment catheter to leave the occlusion apparatus implanted in the body lumen.

In any embodiment, the deployment step comprises:
a first deployment step comprising partially deploying the occlusion apparatus in the body lumen so that the sidewall of the occlusion apparatus engages the body lumen but is not fully deployed and the anchoring arms are partially deployed and not in engagement with the body lumen; and
a second deployment step comprising fully deploying the occlusion apparatus into engagement with the body lumen and fully deploying the anchoring arms so that they are in engagement with the body lumen.

In any embodiment, the method includes a step of treating tissue with the occlusion apparatus after the first deployment step and before the second deployment step.

In any embodiment, the treatment step comprises ablating tissue of the body lumen with tissue ablating elements attached to the occlusion apparatus.

In any embodiment, the method comprises a step of repositioning the occlusion apparatus in the body lumen between the first and second deployment steps.

In any embodiment, the step of repositioning the occlusion apparatus within the body lumen comprises recapturing the occlusion apparatus, and adjusting the position of recaptured occlusion apparatus while it is recaptured.

In any embodiment, the deployment step comprises imaging the occlusion apparatus within the body lumen.

In any embodiment, the occlusion apparatus is at least 80% deployed during the partial deployment while the anchoring arms are not engaged with tissue of the body lumen.

In another aspect, the invention provides a method of capturing embolus in a blood vessel comprising the steps of:
providing a device according to the invention with the embolus capture apparatus and delivery catheter disposed within the deployment catheter;
advancing the device of the invention transluminally until the device is disposed in the blood vessel;
deploying the device by retracting the deployment catheter relative to the delivery catheter to deploy the embolus capture apparatus and anchoring arms;
detaching the delivery catheter from the embolus capture apparatus; and
retracting the delivery catheter and deployment catheter to leave the embolus capture apparatus implanted in the body lumen.

In any embodiment, the deployment step comprises:
a first deployment step comprising partially deploying the embolus capture apparatus in the blood vessel so that the sidewall of the embolus capture apparatus engages the blood vessel but is not fully deployed and the anchoring arms are partially deployed and not in engagement with the blood vessel; and
a second deployment step comprising fully deploying the embolus capture apparatus into engagement with the blood vessel and fully deploying the anchoring arms so that they are in engagement with the blood vessel.

In any embodiment, the method comprises a step of repositioning the embolus capture apparatus in the blood vessel between the first and second deployment steps.

In any embodiment, the step of repositioning the embolus capture apparatus within the blood vessel comprises recapturing the embolus capture apparatus, and adjusting the position of recaptured embolus capture apparatus while it is recaptured.

In any embodiment, the deployment step comprises imaging the embolus capture apparatus within the blood vessel.

In any embodiment, the embolus capture apparatus is at least 80% deployed during the partial deployment while the anchoring arms are not engaged with tissue of the blood vessel.

In any embodiment, the method comprises recapturing the anchoring module and embolus capture apparatus and retracting the recaptured device to remove embolus captured within the embolus capture apparatus.

Other aspects and preferred embodiments of the invention are defined and described in the other claims set out below.

### Brief Description of the Figures

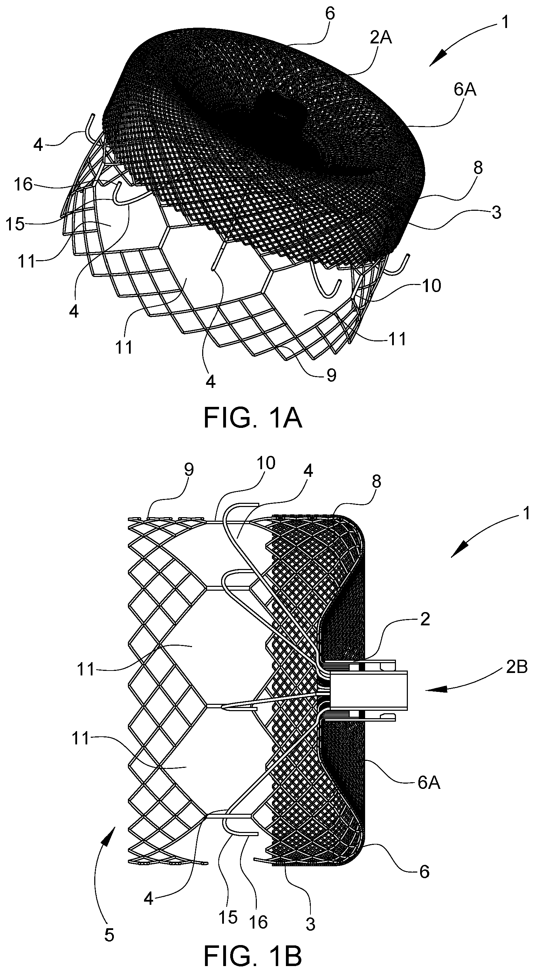

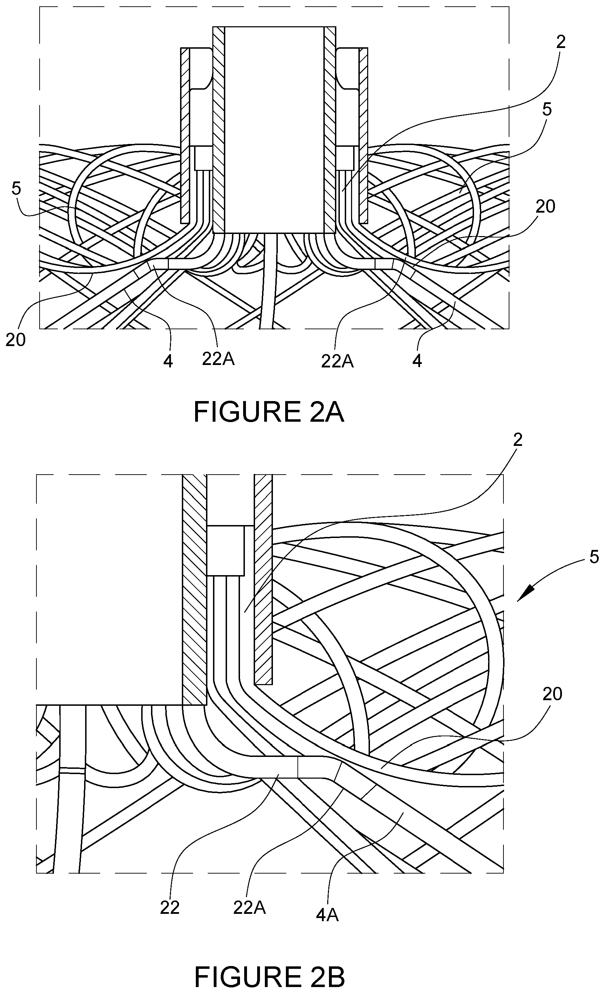

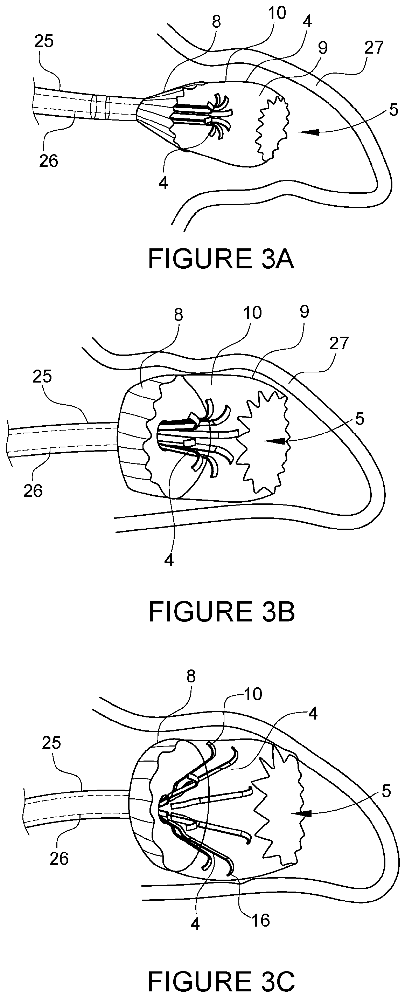

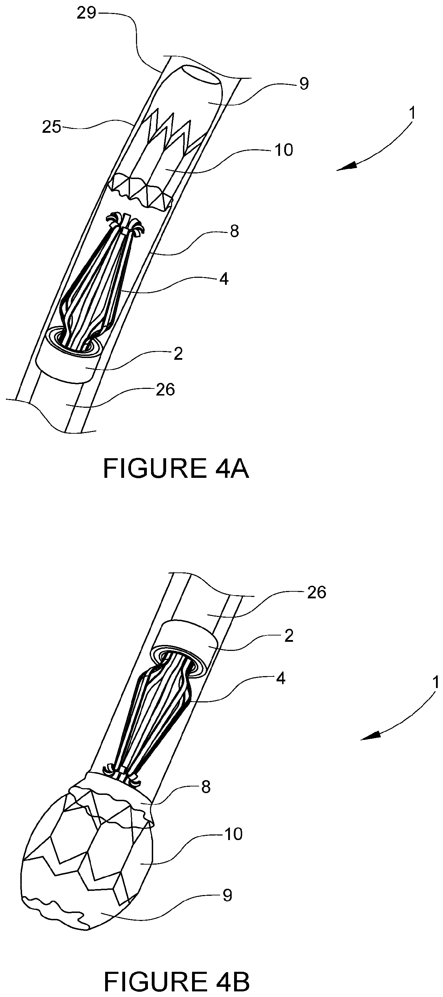

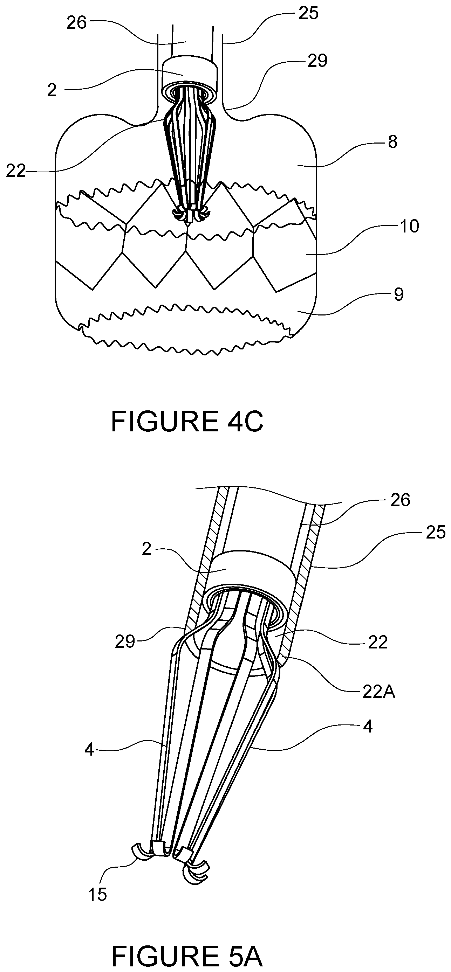

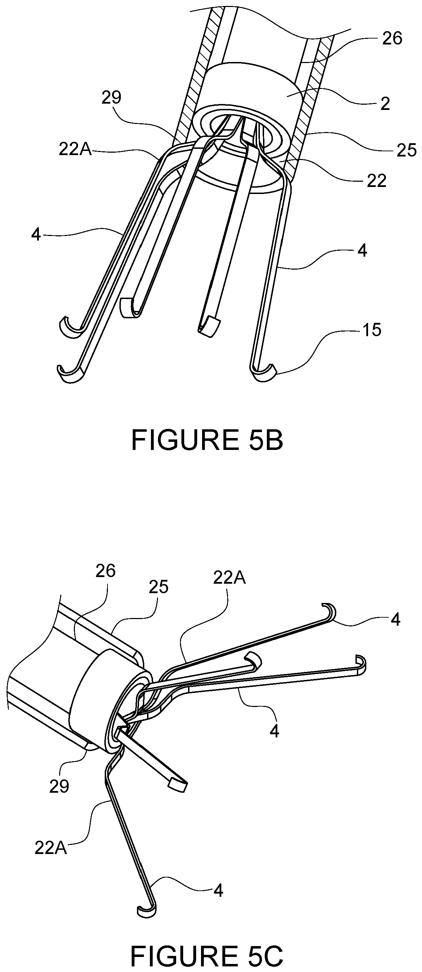

**FIG. 1A** is a perspective view of an occlusion apparatus forming part of the device according to the invention shown in a fully deployed configuration with the ends of the anchoring arms projecting through apertures in a sidewall of the occlusion apparatus. The occlusion apparatus is shown without the delivery catheter or deployment catheter for clarity.
**FIG.1B** is a sectional view of the occlusion apparatus of Figure 1 showing the proximal hub of the occlusion apparatus and radially expansible (e.g. mesh cage) and the anchoring arms attached to the proximal hub and splayed outwardly in a fully deployed configuration at an angle of about 45 to a longitudinal axis of the device.
**FIG'S. 2A and 2B** are detailed views of the device of Figure 1B showing the proximal connecting hub of the occlusion apparatus, the proximal end of the radially expansible cage connected to the connecting hub, and two of the anchoring arms in a fully deployed configuration. This figure shows how the inflection zone (e.g. shoulder) at the proximal end of the anchoring arms cooperates with the proximal end of the radially expansible cage so as to delay full deployment of the anchoring arms until the proximal end of the cage has been deployed, ensuring that the radially expansible cage can be fully or almost fully deployed before the anchoring arms are fully deployed.
**FIG'S 3A to 3C** illustrates a device of the invention being deployed in the left atrial appendage of the heart showing the occlusion apparatus, delivery catheter (broken lines) which is attached to the occlusion apparatus, and outer deployment catheter which is retracted relative to the occlusion apparatus and anchoring arms for deployment of the occlusion apparatus and anchoring arms. Figure 2A shows an initial stage of deployment where the anchoring arms and occlusion apparatus is partially deployed, the arms are within the occlusion apparatus and the occlusion apparatus is not in contact with a wall of the LAA. Figure 2B shows partial deployment of the device where the occlusion apparatus has further deployed to engage the sidewall of the LAA and the anchoring arms have further deployed but not fully and are still within the occlusion apparatus. Figure 2C shows full deployment of the device where the occlusion apparatus and anchoring arms are fully deployed into contact with the wall of the LAA through the apertures in the sidewall of the occlusion apparatus. In Figure 3, the device is anchored in the LAA.
**FIG'S 4A to 3C** are further illustrations of a device according to the invention being deployed, in particular showing self-deployment of the occlusion apparatus and anchoring module by phased retraction of the deployment catheter. Figure 4A shows the device with the occlusion apparatus and anchoring module in a delivery configuration contained within the deployment catheter, and the anchoring arms of the anchoring module is an axial bunched configuration. Figure 4B shows the device during a first stage of partial deployment where the outer deployment catheter has been partially retracted to expose the distal and central sections of the radially expansible body. It can be seen from this figure that at this stage of deployment the radially expansible body has not expended to its full width, and the anchoring arms are constrained into an axial bunched configuration. Figure 4C illustrates the device is a second stage of partial deployment where the deployment catheter has been further retracted (about 90% retracted) exposing most of the radially expansible body. The mouth of the deployment catheter at this stage is in contact with the inflection zone on each anchoring arm keeping the arms in an axial bunched configuration. Further retraction of the deployment catheter at this stage would allow the arms to deploy radially outwardly.
**FIG'S 5A to 5C** are illustrations of the further deployment of the device of Figure 4, in which the radially expansible body has been removed to illustrate more clearly how the anchoring arms deploy in response to retraction of the deployment catheter from the position shown in Figure 4C. In Figure 5A (which is the same stage of deployment as illustrated in Figure 4C), the inflection zone on the arms is in contact with a mouth of the deployment catheter, keeping the arms in an axially bunched configuration. Figure 5B shows how the further retraction of the deployment catheter allows the arms to start to deploy radially outwardly with the deployment controlled by the cooperation between the inflection zone of the arms and the mouth of the deployment catheter. In Figure 5C, further retraction of the deployment catheter fully exposes the inflection zone of the arms proud of the mouth of the deployment catheter allowing the arms to fully self-deploy into engagement with tissue.
**FIG. 6** is an elevational view of a device according to an alternative embodiment of the invention showing an alternative design of anchoring barbs at the distal ends of the anchoring arms.
**FIG. 7** is an elevational view of a device according to an alternative embodiment of the invention showing an alternative design of anchoring barbs at the distal ends of the anchoring arms.
**FIG. 8** is an elevational view of a device according to an alternative embodiment of the invention showing an alternative design of anchoring barbs at the distal ends of the anchoring arms.
**FIG. 9** is a side elevational view of an axially movable anchoring module engaged with an occlusion apparatus with the anchoring arms deployed, and showing the anchoring module hub engaging the proximal hub of the occlusion apparatus and the proximal cover element abutting a proximal face of the radially expansible body.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa*. The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or openended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, age, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the increase in levels of a tight junction protein). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an effective amount or a therapeutically effective amount of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure. Improvement may be observed in biological / molecular markers, clinical or observational improvements. In a preferred embodiment, the methods of the invention are applicable to humans, large racing animals (horses, camels, dogs), and domestic companion animals (cats and dogs).

In the context of treatment and effective amounts as defined above, the term subject (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, camels, bison, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human. As used herein, the term "equine" refers to mammals of the family Equidae, which includes horses, donkeys, asses, kiang and zebra.

"Implantable occlusion apparatus" means an apparatus configured for implantation in a body lumen, especially implantation in the heart at least partially or fully within the left atrial appendage, and upon actuation/deployment to at least partially or fully fluidically occlude the body lumen. The occlusion apparatus is typically detachably connected to a delivery catheter which delivers the occlusion apparatus to the target site, and typically remains attached during occlusion, sensing and energy delivery treatments and in one embodiment is generally detached after the energy delivery treatment and removed from the body leaving the occlusion apparatus implanted in the body lumen. The occlusion apparatus generally includes a central proximal connection hub for attaching to the delivery catheter and a radially expansible body. Occlusion may be complete occlusion (closing) of the body lumen or partial occlusion (narrowing of the body lumen or near complete occlusion). The occlusion apparatus typically comprises a body that is expansible from a contracted delivery configuration to an expanded deployed configuration. The body may take many forms, for example a wireframe structure formed from a braided or meshed material (e.g. a mesh cage). Examples of expandable wireframe structures suitable for transluminal delivery are known in the literature and described in, for example, WO01/87168, US6652548, US2004/219028, US6454775, US4909789, US5573530, WO2013/109756. Other forms of bodies suitable for use with the present invention include plate or saucer shaped scaffolds, or stents. In one embodiment, the body is formed from a metal, for example a shape-memory metal such as nitinol. The body may have any shape suitable for the purpose of the invention, for example cylindrical, discoid or spheroid. In one preferred embodiment, the apparatus comprises a cylindrical body, for example a cylindrical cage body. In one embodiment, the body comprises a tissue energising module. In one embodiment, the ablation device comprises an array of electrodes, typically a circumferential array. In one embodiment, the array of electrodes are configured to deliver pulsed field ablation to the tissue. In one embodiment, a distal face of the radially expansible body comprises a covering configured to promote epithelial cell proliferation. In one embodiment, the body comprises a stepped radial force stiffness profile from distal to proximal device. In one embodiment, the body comprises a metal mesh cage scaffold. In one embodiment, a coupling (e.g. the connecting hub) between the body and the catheter member is located distally to the left atrial facing side of the body. In one embodiment, the body in a deployed configuration has a radial diameter at least 10% greater than the radial diameter of the left atrial appendage at a point of deployment. In one embodiment, the furthermost distal part is configured to be atraumatic to cardiac tissue. In one embodiment, the body comprises a braided mesh scaffold that in one embodiment is conducive to collagen infiltration on thermal energy delivery to promote increased anti migration resistance. Examples of an implantable occlusion apparatus for use in a body lumen especially the LAA are described in WO2018/185256, WO2018/185255 and WO2020/074738.

"Body lumen" means a cavity in the body, and may be an elongated cavity such as a vessel (i.e. an artery, vein, lymph vessel, urethra, ureter, sinus, auditory canal, nasal cavity, bronchus) or an annular space in the heart such as the left atrial appendage, left ventricular outflow tract, the aortic valve, the mitral valve, mitral valve continuity, or heart valve or valve opening.

"Embolus capture apparatus" (or "capture apparatus") refers to a body configured for radial expansion from a contracted delivery configuration to a deployed radially expanded configuration suitable for implantation in a blood vessel. The apparatus is configured to filter blood and capture embolus in the blood. The apparatus generally comprises a radially expansible body configured for deployment in a blood vessel to filter blood and usually comprises a proximal hub. The radially expansible body may take many forms, for example a wireframe structure formed from a braided or meshed material (e.g. a mesh cage). The apparatus may be configured to be deployed in the inferior vena cava. Other forms of bodies suitable for use with the present invention include plate or saucer shaped scaffolds, or stents. In one embodiment, the body is formed from a metal, for example a shape-memory metal such as nitinol. The body may have any shape suitable for the purpose of the invention, for example cylindrical, discoid or spheroid. In one preferred embodiment, the apparatus comprises a cylindrical body, for example a cylindrical cage body. The cage may have an open distal end, open proximal end, or closed distal and proximal ends. Examples of embolus capture apparatus include the SENTRY Bioconvertible Inferior Vana Cava (IVC) filter from Boston Scientific, CELECT Platinum Vena Cava Filter from Cook Medical, and the DENALI Vena Cava Filter from Beckton Dickinson. The use of embolus capture apparatus is described in https://www.drugwatch.com/ivc-filters/.

"Detachably attached" means that the device is configured such that the occlusion apparatus or capture apparatus is attached to the elongated delivery catheter during delivery and can be released after deployment and treatment whereby the apparatus is implanted in the heart and the elongated delivery catheter can be withdrawn leaving the apparatus in-situ. Typically, the device includes a control mechanism for remotely detaching the apparatus or radially expansible element from the elongated catheter member. Typically, an actuation switch for the control mechanism is disposed on the control handle.

"Transluminal delivery" means delivery of the occlusion apparatus or capture to a target site (for example the heart) heart through a body lumen, for example delivery through an artery or vein. In one embodiment, the device of the invention is advanced through an artery or vein to deliver the occlusion apparatus to the left atrium of the heart and at least partially in the LAA. In one embodiment, the device is delivered such that the distal part is disposed within the LAA and the proximal part is disposed in the left atrium just outside the LAA. In one embodiment, the device is delivered such that the distal part is disposed within the LAA and the proximal part is disposed in the left atrium abutting a mouth of the LAA. In one embodiment, the device is delivered such that both the distal and proximal parts are disposed within the LAA.

"Anchoring module" means an anchoring arm, and preferably an array of anchoring arms that can be deployed to anchor the occlusion apparatus or capture apparatus in the body lumen. The anchoring arms may be made from a shape memory material, such as nitinol. The anchoring arms are generally adjustable (e.g. pivotally adjustable about their proximal end) from an axial position prior to deployment (in which the anchoring arms are generally bunched together along or close to a longitudinal axis of the device) to an outwardly splayed configuration. When fully deployed a distal end of at least some of the anchoring arms generally extend through apertures in the radially expansible body to engage tissue. The anchoring arms are generally biased into the outwardly splayed configuration and deployed by releasing a constraining member, such as a deployment catheter. This is also referred to herein as self-deployment. In some of the embodiments described herein, the anchoring arms are attached to the proximal hub of the occlusion or capture apparatus. The anchoring module is generally not movable radially relative to the radially expansible body. In another embodiment, the anchoring module is movable relative to the occlusion or capture apparatus. For example, the anchoring module may be attached to an anchoring catheter and movable axially through (e.g. the delivery catheter) and the lumen in the proximal connecting hub to deliver the anchoring module into the radially expansible body. The anchoring module may comprise an central lumen

. The proximal connecting hub and hub of the anchoring module may be configured for detachable attachment, providing the anchoring arms inside the radially expansible body for deployment therewith. The anchoring module may include at least 2, 3, 4, 5 or 6 anchoring arms. In any embodiment, one or more of the anchoring arms comprise a tissue treatment element such as a tissue ablation electrode.

"Inflection zone" refers to a part of a proximal section of an anchoring arm that is shaped to cooperate with a proximal end of the radially expansible body during deployment to delay full deployment of the anchoring arm until the proximal end of the radially expansible body has been deployed out of the deployment catheter. It generally includes a radially outward shoulder. It may include a proximal inflection zone which curves radially outwardly and a distal inflection zone which curves radially inwardly (for example a s-shaped section). Alternatively, the proximal part of the arm may be cranked intermediate its ends.

"Cover": Typically, the implantable occlusion apparatus has a proximal cover which is impermeable to blood and that may include a re-closable aperture, for example an overlapping flap of material. The re-closable aperture may be configured to allow a distal end of the catheter through the aperture while preventing blood flow through the aperture. The occlusion apparatus may include a connecting hub distal of the cover, and configured for coupling with a distal end of the catheter. The cover may be configured to act as a scaffold for in-vivo endothelialisation. The cover may be formed from a woven mesh material.

"Covering/cover configured to act as a scaffold for in-vivo endothelialisation" means a material that is use promotes epithelialisation of the distal or proximal body. In one embodiment, the covering is a membrane that comprises agents that promote epithelial cell proliferation. Examples include growth factors such as fibroblast growth factor, transforming growth factor, epidermal growth factor and platelet derived growth factor, cells such as endothelial cells or endothelial progenitor cells, and biological material such as tissue or tissue components. Examples of tissue components include endothelial tissue, extracellular matrix, sub-mucosa, dura mater, pericardium, endocardium, serosa, peritoneum, and basement membrane tissue. In one embodiment, the covering is porous. In one embodiment, the covering is a biocompatible scaffold formed from biological material. In one embodiment, the covering is a porous scaffold formed from a biological material such as collagen. In one embodiment, the covering is a lyophilised scaffold.

The device of the invention may include a tissue energising module. "Tissue energising module" as used herein refers to an array of tissue treating elements configured to treat tissue by application of, e.g., heat, cold, sound, light, microwave energy, or RF energy. The elements may be electrodes. The electrodes disposed on the implantable occlusion apparatus configured for electrical coupling with the electrical controller. The electrodes are generally individually coupled with the controller to allow electrode specific energising of the electrode. They array of electrodes is generally arranged on the implantable apparatus in a circumferential arrangement and configured to contact the wall of the body lumen in a circumferential pattern when the apparatus is deployed. The electrodes are configured to deliver energy, generally PFA, circumferentially around the wall of the body lumen. The electrodes may also function as sensors to detect an electrical parameter of the tissue of the wall of the body lumen, for example electrical impedance or electrical activity (voltage), or electrical mapping of the LAA or heart. The electrodes may be configured to measure an electrical parameter radially across the wall of the body lumen, or circumferentially along a section of the circumference of the wall of the body lumen. Generally, measuring an electrical parameter such as electrical impedance radially across the wall of the body lumen employs an electrode of the array of electrodes and an earth or ground pad placed on the patient's body, often the leg. Measuring an electrical parameter such as electrical impedance circumferentially along a section of the body lumen employs two electrodes where one electrode functions as an energising electrode and the other functions as a detecting electrode. The electrical parameter such as electrical impedance may be measured at one frequency or over a range of frequencies.

The device of the invention may be used to prevent or treat or diagnose a cardiac condition such as atrial fibrillation. The invention may also relate to a method of preventing or treating or diagnosing atrial fibrillation. "Atrial fibrillation" or "AF" is a common cardiac rhythm disorder affecting an estimated 6 million patients in the United States alone. AF is the second leading cause of stroke in the United States and may account for nearly one-third of strokes in the elderly. In greater than 90% of cases where a blood clot (thrombus) is found in the AF patient, the clot develops in the left atrial appendage (LAA) of the heart. The irregular heartbeat in AF causes blood to pool in the left atrial appendage, because clotting occurs when blood is stagnant, clots or thrombi may form in the LAA. These blood clots may dislodge from the left atrial appendage and may enter the cranial circulation causing a stroke, the coronary circulation causing a myocardial infarction, the peripheral circulation causing limb ischemia, as well as other vascular beds. The term includes all forms of atrial fibrillation, including paroxysmal (intermittent) AF and persistent and longstanding persistent AF (PLPAF).

The device of the invention may be used to prevent or treat or diagnose a cardiac condition such as an ischaemic event. The invention may also relate to a method of preventing or treating or diagnosing an ischaemic event. "Ischaemic event" refers to a restriction in blood supply to a body organ or tissue, resulting in a shortage of oxygen and glucose supply to the affected organ or tissue. The term includes stroke, a blockage of blood supply to a part of the brain caused by a blood clot blocking the blood supply to the brain and the resultant damage to the affected part of the brain, and transient ischaemic events (TIA's), also known as "mini-strokes", which are similar to strokes but are transient in nature and generally do not cause lasting damage to the brain. When the restriction in blood supply occurs in the coronary arteries, the ischaemic event is known as a myocardial infarction (MI) or heart attack.

The occlusion apparatus or capture apparatus may be self-deployable. The radially expansible body may be self-deployable. At least one of the anchoring arms may be self-deployable. The occlusion body may be made from a shape memory material. The radially expansible body may be made from a shape memory material. At least one of the anchoring arms may be self-deployable. The anchoring module may be movable axially relative to the apparatus. The anchoring module may be rotatable relative to the apparatus about a longitudinal axis of the device. The apparatus may be rotatable about a longitudinal axis of the device. The anchoring module may be fixed to the apparatus. The apparatus when deployed may have a lateral dimension (width) at least 10%, 15%, 20% or 25% greater than a width of the body lumen to be treated. The radially expansible body may be radially expansible to a width of the body lumen without full deployment of the anchoring arms. A proximal end of the radially expansible body may be configured to cooperate with a proximal end of at least one anchoring arm during deployment of the device to retain the anchoring arms within the radially expansible body until the radially expansible body has been deployed fully.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

Referring to the drawings and initially to Figures 1A and 1B, there is illustrated a first embodiment of an occlusion apparatus forming part of a device according to the invention indicated generally by the reference numeral 1. The occlusion apparatus is shown in a fully deployed configuration, and the deployment catheter and delivery catheter are not shown. The occlusion apparatus comprises a proximal connection hub 2 having an internal lumen 2B, a radially expansible body (in this case a mesh cage 3), and an anchoring module comprising a circumferential array of anchoring arms 4 attached to the proximal connection hub. In this embodiment, the anchoring module has ten arms.

The mesh cage 3 is cylindrical when deployed with an open distal end 5 and closed proximal end 6 having a concave recess 6A. A proximal part 2A of the proximal connecting hub 2 is disposed in the recess 6A of the proximal end 5 of the mesh cage 3. Although not shown, a fluid impermeable cover member will be fitted over the proximal end of the cage to prevent access of blood to the proximal connecting hub, the cover member including a closable aperture allowing a delivery catheter access the recess 6A to connect with the proximal connection hub 2.

The mesh cage 3 has three sections, a proximal section 8 having a small mesh size, for example about 2.5 mm, a distal section 9 having a mesh size of about 2.5 mm, and a central section 10 having apertures 11 for receiving the anchoring arms during deployment. The apertures 11 are sufficiently large to prevent the ends of the anchoring arms snag on the mesh during deployment; in the embodiment shown, the apertures have an axial length of about 5 mm and an width of about 8 mm. The mesh cage is made from nitinol, a shape memory material, and is configured to radially expand to the configuration shown when it is deployed.

Generally deployment comprises retraction of a constraining deployment catheter to release the mesh cage where it expands into contact with the wall of the body lumen in which is it positioned. Generally, the radially expansible body (e.g. mesh cage is configured to be oversized when deployed relative to the body lumen in which it is to be deployed, for example oversized by about 5-30% and more specifically about 15-20%.

The radially expansible element is also designed to fully, or almost fully, deploy laterally while it has not been fully released from the deployment catheter. This is illustrated in Figure 3B which shows the partially deployed cage (about 80% of which has been released from the deployment catheter) in almost full lateral deployment and contacting the walls of the body lumen while the arms are not deployed and not in contact with the tissue. The advantages of this arrangement are that is allows a phased deployment of the device including a first deployment stage where the cage is deployed into contact with the wall of the body lumen with the anchoring arms not fully deployed and the device therefore not anchored. This allows the positioning of the device to be assessed (e.g. by imaging). If the device positioning is determined to be sub-optimal, the device can be recaptured and re-positioned and then partially deployed again, its positioning checked, and then fully deployed if the positioning is determined to be correct where the anchoring arms are fully deployed into contact with the tissue to anchor the device in the body lumen.

The anchoring arms 4 are formed from nitinol and are biased into the outwardly splayed position shown in Figure 1B, allowing deployment when the constraining deployment catheter is retracted. The arms are connected to the hub 2 of the occlusion apparatus and are not connected to the radially expansible body (mesh cage), allowing flexibility for the arms to move independently of the mesh cage. Each arm 4 has a proximal end 4A and a distal end 4B. This distal end 4B is curved outwardly and proximally forming a hook-shaped barb 15 having a tip 16 which faces proximally and is substantially parallel to a longitudinal axis of the device.

This is advantageous as devices in the LAA tend to be pulled proximally (towards the left atrium), so the backward-facing barbs help prevent this.

Figures 2A and 2B illustrate a proximal end of the occlusion apparatus 1 showing the proximal connecting hub 2, proximal recessed end 6 of the mesh cage, and proximal ends 4A of the anchoring arms 4. The struts 20 of the proximal end of the cage are attached to a radially outward part A of the connecting hub 2, and the anchoring arms 4 are attached to a radially inward part B of the hub 2. As illustrated in Figure 2B, the proximal end 4A of the anchoring arms have an inflection zone 22 forming a shoulder 22A. The shoulder 22A cooperates with the struts 20 of the proximal end of the cage, to control the deployment of the arms. Thus, full deployment of the arms is delated until the proximal end of the cage is released from the deployment catheter and the cage is fully deployed. This allows the phased deployment of the device as described above and below, maintaining the anchoring arms within the cage until the cage has been fully released from the deployment catheter.

Figures 3A to 3C further describe one embodiment of the device of the invention and its use, in particular the phased deployment and anchoring of the device in a body lumen, in this case the left atrial appendage (LAA) of the heart. Figure 3A shows the device of the invention comprising occlusion apparatus 1, outer deployment catheter 25 and inner delivery catheter 26 (shown in broken lines). The device is shown with a distal end disposed in the left atrial appendage 27, and with about 50% of the radially expansible body 3 deployed out of a distal end of the deployment catheter and partially deployed to about 60% of its full width. In Figure 3B, the deployment catheter has been further retracted relative to the occlusion device, so that the radially expansible body is about 80% deployed out of a distal end of the deployment catheter with almost full radial expansion so that the walls of the radially expansible body engage the walls of the LAA. At this stage, it can be seen that the anchoring arms 4 are not fully splayed outwardly and are not in engagement with this tissue. A contrast dye may be injected into the patient to image the position of the device in the LAA. Further test can also be performed to determine the suitability of the positioning of the device. If the cardiologist is not satisfied with the positioning, the device can be re-captured and re-positioned.

Alternatively, the device can be used to electrically ablate the tissue. Once the cardiologist is satisfied that the radially expansible body has been correctly positioned, and as illustrated in Figure 3B, the device is actuated to further retract the deployment catheter 25 relative to the delivery catheter 26, to fully deploy the radially expansible body 3 and anchoring arms which engage the wall of the LAA to anchor the device in place. Further ablative treatments can them be performed. Once treatment is finished, the delivery catheter 26 may be actuated to detach from the hub 2 of the occlusion apparatus and withdrawn along with the deployment catheter 25 to leave the occlusion apparatus anchored in-situ in the LAA of the heart.

The device may include a control handle configured to move the deployment catheter relative to the delivery catheter and/or detach or attach the delivery catheter and the occlusion body. The control handle may be configured to actuate deployment of the radially expansible body independently of the anchoring arms. The device may also include tissue ablation electrodes, generally formed as part of, or attached to, the radially expansible body. Electrical leads may be provided to electrically connect the electrodes with corresponding electrical leads provided in the delivery catheter. The connecting hub of the occlusion apparatus and of the delivery catheter may be configured to electrically couple the electrodes of the radially expansible body with the electrical leads of the delivery catheter.

FIG'S 4A to 4C are further illustrations of a device according to the invention being deployed, in particular showing self-deployment of the occlusion apparatus and anchoring module by phased retraction of the deployment catheter. Figure 4A shows the device with the occlusion apparatus 1 and anchoring module in a delivery configuration contained within the deployment catheter 25, and the anchoring arms 4 of the anchoring module is an axial bunched configuration. Figure 4B shows the device during a first stage of partial deployment where the deployment catheter 25 has been partially retracted to expose the distal 9 and central sections 10 of the radially expansible body 3. It can be seen from this figure that at this stage of deployment the radially expansible body 3 has not expended to its full width, and the anchoring arms 4 are constrained into an axial bunched configuration. Figure 4C illustrates the device in a second stage of partial deployment where the deployment catheter 25 has been further retracted (about 90% retracted) exposing most of the radially expansible body 3. A mouth/lip 29 of the deployment catheter 25 at this stage is in contact with the inflection zone 22 on each anchoring arm 4 keeping the arms in an axial bunched configuration. Further retraction of the deployment catheter at this stage would allow the arms to deploy radially outwardly.

FIG'S 5A to 5C are illustrations of the further deployment of the device of Figure 4, in which the radially expansible body has been removed to illustrate more clearly how the anchoring arms deploy in response to retraction of the deployment catheter from the position shown in Figure 4C. In Figure 5A (which is the same stage of deployment as illustrated in Figure 4C), the inflection zone 22 on the arms 4 is in contact with a mouth/lip 29 of the deployment catheter, keeping the arms in an axially bunched configuration. Figure 5B shows how the further retraction of the deployment catheter 25 allows the arms 4 to start to deploy radially outwardly with the deployment controlled by the cooperation between the inflection zone 22 of the arms 4 and the mouth/lip 22 of the deployment catheter. In Figure 5C, further retraction of the deployment catheter 25 fully exposes the inflection zone 22 of the arms proud of the mouth 29 of the deployment catheter allowing the arms to fully self-deploy into engagement with tissue.

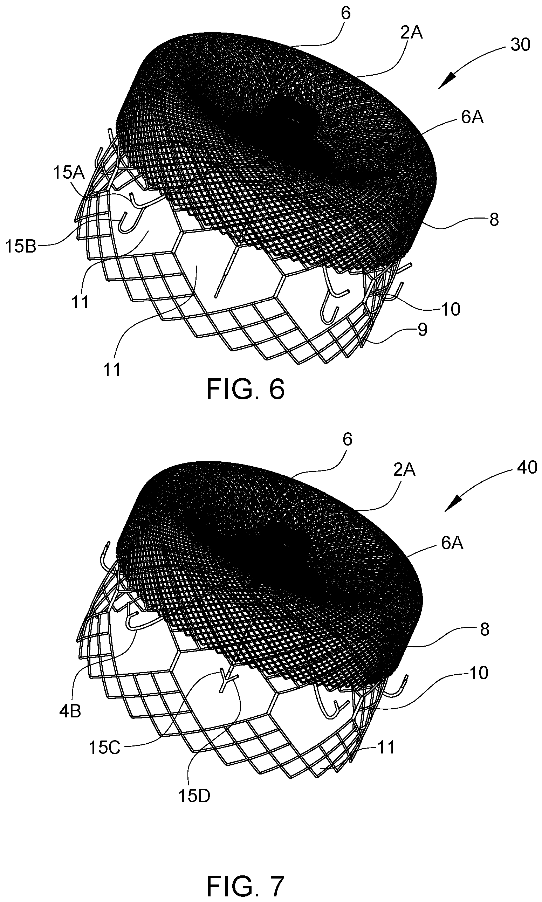

Referring to Figure 6, an occlusion apparatus forming part of a device according to an alternative embodiment of the invention is shown, indicated generally by the reference numeral 30, and in which parts described with reference to the previous embodiments are assigned the same reference numerals. In this embodiment, the distal end 4B of the anchoring arms is birfurcated longitudinally to provide a u-shaped distal barb 15A and a curved proximal barb 15B. Provision of two barbs, one distal and one proximal, has been shown to increase the chances that at least one barb per arm will engage the tissue, thereby reducing the risk of device migration.

Referring to Figure 7, an occlusion apparatus forming part of a device according to an alternative embodiment of the invention is shown, indicated generally by the reference numeral 40, and in which parts described with reference to the previous embodiments are assigned the same reference numerals. In this embodiment, the distal end 4B of the anchoring arms is birfurcated transversely to provide a v-shaped barb having first and second barb parts 15C and 15D. Provision of two side-by-side barbs, has been shown to increase the chances that at least one barb per arm will engage the tissue, thereby reducing the risk of device migration.

Referring to Figure 8, an occlusion apparatus forming part of a device according to an alternative embodiment of the invention is shown, indicated generally by the reference numeral 50, and in which parts described with reference to the previous embodiments are assigned the same reference numerals. In this embodiment, the distal end 4B of each anchoring arm is cranked intermediate its end so that when it is fully deployed, it has a first part 41 that is angled radially outwardly, a second part 42 that is disposed inside the deployed radially expansible body 3 parallel with a longitudinal axis of the device, and an outer u-shaped hook 15E. The barb strut goes parallel to the scaffold before ethe barb hooks back. This provides a flat section pushing against the tissue or scaffold limiting the amount of perforation into the tissue.

Referring to Figure 9, an embolus capture apparatus forming part of a device according to an alternative embodiment of the invention is shown, indicated generally by the reference numeral 60, and in which parts described with reference to the previous embodiments are assigned the same reference numerals. In this embodiment, the anchoring module is axially movable relative to the embolus capture apparatus 60 and comprises anchoring arms 4 attached to central anchoring hub 61. The central anchoring hub 61 is configured to be received within and detachable engage with the connecting hub 2 of the embolus capture apparatus 1. The anchoring module additionally includes an annular cover element 62 that extends radially outwardly from the hub and has a slightly convex shape configured to fluidically engage with the concave proximal face 6 of the cage 3 to prevent embolus passing through connecting hub 2. The annular cover element 62 is self-adjustable from a contracted delivery configuration to the deployed radially expanded configuration shown in Figure 9. The cover element may be formed from a mesh, for example a nitinol mesh, designed to self-deploy when a restraining element such as a delivery catheter is retracted proximally relative to the anchoring module and has a mesh size dimensioned to allow blood through but to capture embolus. In other embodiments, the cover element may be formed for a number of elements connected to the central anchoring hub 61 that are adjustable from an axial position where the elements are bunched together and a deployed position where the elements extend radially outwardly and overlap to form the cover element. In other embodiments, the anchoring module does not include a cover element. In such embodiments, the engagement between the anchoring module hub and occlusion apparatus hub may be a fluidically tight engagement that effectively closes the hub of the occlusion apparatus. In use, the embolus capture apparatus may be first deployed and, while the delivery catheter is still attached to the proximal hub of the embolus capture apparatus, the anchoring module is advanced though the delivery catheter with the anchoring arms in an axially bunched together delivery configuration. The arms are advanced through the hub of the deployed embolus capture apparatus until the anchoring module hub engages the hub of the embolus capture apparatus. In this position, the arms will self-deploy into the embolus capture cage into contact with the surrounding tissue to anchor the embolus capture apparatus in the blood vessel. The delivery catheter (not shown in Figure 9) may then be detached from the hub of the embolus capture apparatus and retracted, allowing the cover element of the anchoring module self-deploy into the radially expanded configuration shown in Figure 9, to cover the hub of the occlusion apparatus and prevent embolus moving proximally through the embolus capture apparatus. It will be appreciated that while this embodiment is described with reference to an embolus capture apparatus, an axially movable anchoring module (with or without an annular cover element) may also be used with a body lumen occlusion apparatus, in which case the cover if employed will be configured to fluidically occlude the hub of the occlusion apparatus to prevent blood flowing distally through the hub.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A device to occlude a left atrial appendage of a heart of a subject, comprising:
an implantable occlusion apparatus comprising a proximal hub and a radially expansible part configured for radial expansion upon deployment from a contracted configuration to a radially expanded configuration to fluidically occlude the left atrial appendage;
an elongated delivery catheter having a distal connecting hub attachable to the proximal hub of the implantable occlusion apparatus for transluminal delivery of the implantable occlusion apparatus to the left atrial appendage; and
an anchoring module comprising a circumferential array of anchoring arms configured for pivotable self-adjustment from a delivery configuration in which the array of anchoring arms are arranged axially and a deployed configuration in which the anchoring arms are splayed radially outwardly to engage a wall of the left atrial appendage through an open section of a sidewall of the occlusion apparatus,
wherein a distal end of one or more of the anchoring arms comprises a distal anchoring barb configured to engage tissue and a proximal anchoring barb configured to engage tissue

2. A device according to claim 1, in which each anchoring arm is connected to and extends distally from a proximal hub of the occlusion apparatus and is movable independently of the radially expansible part.

3. A device according to Claim 1, in which the anchoring arms are self-adjustable independently of each other.

4. A device according to any preceding claim, in which the device is configured for adjustment from a partially deployed configuration in which the sidewall of the radially expansible part engages a wall of the left atrial appendage and the anchoring arms are not engaged with the wall of the left atrial appendage and a fully deployed configuration in which the anchoring arms are engaged with the wall of the left atrial appendage to anchor the occlusion apparatus or capture apparatus in the body lumen.

5. A device according to any preceding Claim 1, in which one or more of the anchoring arms has a proximal section and a distal section, in which the proximal section has an inflection zone configured to cooperate with a proximal end of the radially expansible part during deployment of the radially expansible part to deploy the one or more of the anchoring arms into a partially deployed configuration and, upon further deployment of the radially expansible part, into the fully deployed configuration.

6. A device according to claim 5, in which the inflection zone of the arm comprises an s-shaped section comprising a proximal inwardly curved part and a distal outwardly curved part.

7. A device according to any preceding Claim, in which each anchoring arm in a deployed configuration is splayed radially outwardly at an angle of 30-80° to a central axis of the implantable occlusion apparatus.

8. A device according to any preceding Claim, in which the anchoring arms have an axial length that is less than 70% of an axial length of the cage.

9. A device according to any preceding Claim, in which the radially expansible part comprises a mesh cage.
